# EUROPÄISCHE PATENTSCHRIFT

(11) **EP 0 961 624 B1**
(45) Veröffentlichungstag und Bekanntmachung des Hinweises auf die Patenterteilung: **04.08.2004**
(21) Anmeldenummer: 96913463.4
(22) Anmeldetag: 03.05.1996
(51) Int. Cl.: A61M 5/142, A61M 5/168

(54) **IMPLANTIERBARE INFUSIONSPUMPE**
IMPLANTABLE DIFFUSION PUMP
POMPE A PERFUSION IMPLANTABLE

(30) Priorität: 03.05.1995 DE 19515722
(43) Veröffentlichungstag der Anmeldung: 08.12.1999
(73) Patentinhaber: Codman Neuro Sciences Sàrl, 2400 Le Locle (CH)
(72) Erfinder: OTTO, Karl-Heinz, D-24146 Kiel (DE); WIELAND, Manfred, D-24146 Kiel (DE); BAUMANN, Hans, D-24223 Raisdorf (DE); PETERS, Jörg-Roger, D-24582 Bordelsholm (DE)
(74) Vertreter: Bardehle, Heinz, Dipl.-Ing.
(86) Internationale Anmeldenummer: PCT/DE1996/000771
(87) Internationale Veröffentlichungsnummer: WO 1996/034639

(56) Entgegenhaltungen:
- EP-A- 0 189 940
- EP-A- 0 369 712
- US-A- 3 921 916
- US-A- 4 537 680
- US-A- 5 176 360

## Beschreibung

Die Erfindung betrifft eine implantierbare Infusionspumpe, mit einem Gehäuse, in dem ein ein einen Dampfdruck erzeugendes Treibmittel aufnehmender Treibmittelraum und ein ein Medikament aufnehmender Medikamentenraum ausgebildet sind, und mit einer die Abgabe des Medikament dosierenden, zu einem im Körper des Patienten mündenden Katheter führenden Drosselstrecke, die als Chip mit einer in diese eingeätzten Fluidbahn ausgebildet ist.

Eine derartige Infusionspumpe ist aus der EP 189 940 bekannt.

Der Erfindung liegt die Aufgabe zugrunde, eine Infusionspumpe mit einem als Drosselstrecke dienenden Chip zu schaffen, bei dem einerseits die Konnektion des Einlasses- und des Auslasses zuverlässig und gegen auftretende Stoßbelastungen widerstandsfähig ist.

Erfindungsgemäß wird diese Aufgabe durch die Merkmale des Anspruchs 1 gelöst.

Die Erfindung wird im folgenden anhand einer Zeichnung erläutert. Dabei zeigt:
- Fig. 1: eine Schnittansicht eines in eine nur teilweise dargestellte Infusionspumpe eingesetzten Chip, und
- Fig. 2: eine Draufsicht auf die obere Fläche des Chips.

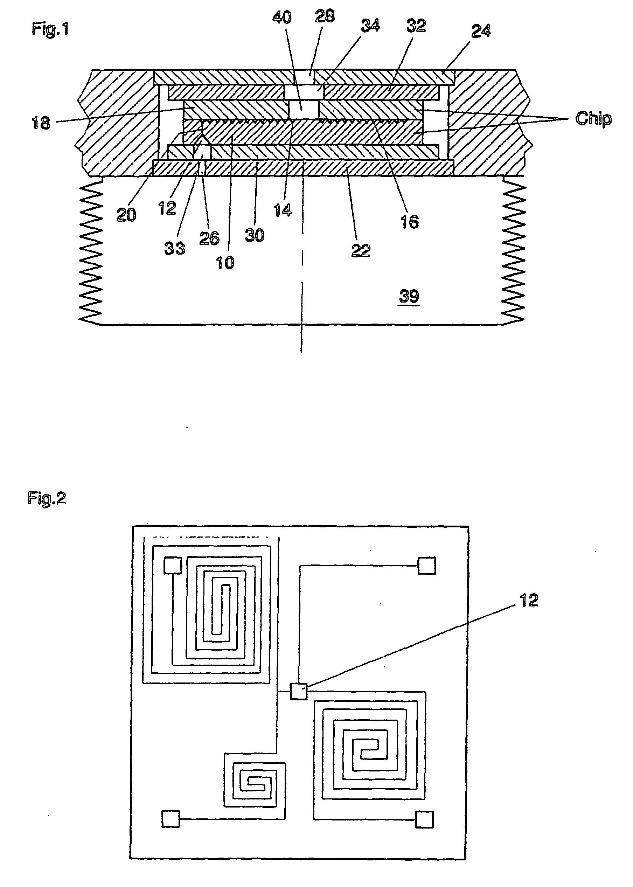

In Figur 1 ist ein Teil der Infusionspumpe in einer Querschnittsdarstellung gezeigt.

Es ist erkennbar, daß die obere Abdeckung des Medikamentenraumes 39 mit einer Ausnehmung versehen ist, in der ein Chip 10 angeordnet ist. Die obere Fläche des Chips 10 ist mit einer Mehrzahl von mäanderförmig verlaufenden Fluidbahnen 16 versehen, die einen Einlaß 12 mit einem Auslaß 14 verbinden. Diese Fluidbahnen 16 sind durch ein photolithographisches Verfahren, wie es zur Herstellung von Halbleiterschaltungen bekannt ist, in die obere Fläche des Chips 10 geätzt. Eine aus einem transparenten Glas bestehende Abdeckung 18 liegt auf der oberen Fläche des Chips 10 auf und begrenzt so die Fluidbahnen 16 nach oben.

Der Chip 10 ist nach unten mit einer ersten Silikonschicht 30 versehen, auf der Abdeckung 18 liegt eine zweite Silikonschicht 32 auf. Dieser Aufbau aus Chip 10, Abdeckung 18 und Silikonschichten 30 und 32 wird oben und unten von metallischen Plättchen 22, 24 eingefaßt, über die die Anordnung in der oberen Abdeckung der Medikamentenkammer 39 befestigt, vorzugsweise lasergeschweißt, ist. Dabei dienen die Silikonschichten 30, 32 zum Auffangen von Stößen, um den Chip 10 und die Abdeckung 18 vor mechanischen Stößen zu schützen.

Das untere metallische Plättchen 22 ist mit einer Bohrung 26, die Silikonschicht 30 mit einer Ausnehmung 32 versehen. Die Bohrung 26 und die Ausnehmung 33 fluchten mit dem Einlaß 12 des Chips 10, der über einen durch den Chip hindurchgeführten Kanal 20 zu der Fluidbahn 16 in der oberen Fläche des Chips 10 führt.

Die Abdeckung 18 weist eine zentrale Bohrung 40 auf, die mit dem ebenfalls zentral angeordneten Auslaß 14 der Fluidbahn 16 fluchtet. Die obere Silikonschicht 32 ist mit einer Ausnehmung 34, das obere metallische Plättchen 24 mit einer Bohrung 28 versehen, die ebenfalls mit dem Auslaß 14 der Fluidbahn fluchten.

Das von dem Medikamentenraum 39 aufgenommene Medikament kann auf diese Weise durch den Kanal 20 und die Fluidbahn 16 hindurch nach oben durch die Bohrung 28 in dem oberen metallischen Plättchen 24 austreten, an der ein - nicht dargestellter - Anschluß zu einem Katheter vorgesehen ist.

Figur 2 verdeutlicht, daß bei einer quadratischen Ausbildung des Chips 10 der Eingang 12 mittig angeordnet ist, während vier Ausgänge 14 benachbart den vier Ecken des Chips in gleichem Abstand von der jeweiligen Ecke angeordnet sind. Dies ermöglicht durch eine geeignete Montage auszuwählen, welche der vier ausgebildeten Drosselstrecken aktiv ist. Auf diese Weise kann der Drosselwiderstand den jeweiligen Anforderungen entsprechend gewählt werden.

## Patentansprüche

1. Implantierbare Infusionspumpe, mit einem Gehäuse, in dem ein ein einen Dampfdruck erzeugendes Treibmittel aufnehmender Treibmittelraum und ein ein Medikament aufnehmender Medikamentenraum ausgebildet sind, und mit einer die Abgabe des Medikaments dosierenden, zu einem im Körper des Patienten münden Katheter führenden Drosselstrecke, die als Chip (10) ausgebildet ist, in dessen obere Fläche durch ein fotolitographisches Verfahren eine mit einem Einlass (12) und einem Auslass (14) versehene Fluidbahn (16) eingeätzt ist, wobei die obere Fläche des Chips (10) mit einer aufgebondeten, hochebenen Abdeckung (18) versehen ist, wobei der Einlass (12) und der Auslass (14) als in die Fläche eingeätzte Vertiefungen ausgebildet sind, und die den Einlass (12) bildende Vertiefung mit einem zu der Rückfläche des Chips führenden Kanal (20) versehen ist, **dadurch gekennzeichnet, dass** die obere Fläche und die untere Fläche des Chips mit einem metallischen Plättchen (22, 24) abgedeckt sind, die mit mit dem Einlass (12) bzw. dem Auslass (14) fluchtenden Bohrungen (26, 28) versehen ist, wobei zwischen der Fläche des Chips und den Metallplättchen (22, 24) Silikonschichten (30, 32) angeordnet sind, die mit einer mit dem Einlass (12) bzw. dem Auslass (14) fluchtenden Ausnehmung (34, 36) versehen ist.

2. Implantierbare Infusionspumpe nach Anspruch 1, **dadurch gekennzeichnet, dass** auf dem Chip eine Mehrzahl von einen unterschiedlichen Drosselwiderstand darstellende Fluidbahnen ausgebildet sind, wobei der Einlass (12) zentral angeordnet ist und die Auslässe (14) der einzelnen Fluidbahnen zu dem Einlass (12) punktsymmetrisch angeordnet sind.

## Claims

1. Implantable infusion pump, with a housing, in which are formed a blowing agent room which accommodates a blowing agent generating a vapor pressure and a medicament room which accommodates a medicament, and with a regulator line which doses the dispersion of said medicament and which guides to a catheter meeting in the body of a patient, wherein said regulator line is formed as chip (10) in which upper area a fluid channel (16) provided with an inlet (12) and an outlet (14) is etched by a photo lithographic method, wherein said upper area of said chip (10) is provided with a stuck on, even cover (18), wherein said inlet (12) and said outlet (14) are formed as indentation etched into the area and said indentation forming said inlet (12) is provided which a channel (20) guiding to the rear area of the chip, **characterized in that** said upper area and said lower area of said chip are covered with a metal lamina (22, 24) which is provided with borings (26, 28) aligned with said inlet (12) and said outlet (14), respectively, wherein between said area of said chip and said metal laminas silicon layers (30, 32) are formed, which are provided with recesses (34, 36) aligned with said inlet (12) and said outlet (14), respectively.

2. Implantable infusion pump according to claim 1, **characterized in that** on said chip a plurality of fluid channels are formed representing different regulator resistant, wherein said inlet (12) is arranged centrally and said outlets (14) of the respective fluid channels are arranged point symmetrical to said inlet (12).

## Revendications

1. Pompe implantable à perfusion, comportant un boîtier, dans lequel sont formés un compartiment de fluide propulseur, qui reçoit un fluide propulseur produisant une pression de vapeur, et un compartiment de médicament, qui reçoit un médicament, et comportant une voie d'étranglement, qui sert à doser le débit du médicament et qui conduit à un cathéter débouchant dans le corps du patient, laquelle voie d'étranglement est réalisée en tant que puce (10), dans la face supérieure de laquelle est constitué, par attaque chimique au moyen d'un procédé de photolithographie, un chemin d'écoulement de fluide (16) pourvu d'une entrée (12) et d'une sortie (14), la face supérieure de la puce (10) étant recouverte d'un revêtement formant plateau (18) fixé par collage, l'entrée (12) et la sortie (14) étant réalisées en tant que cavités pratiquées par attaque chimique dans la face, et la cavité constituant l'entrée (12) étant pourvue d'un canal (20) menant vers la face arrière de la puce, **caractérisée en ce que** la face supérieure et la face inférieure de la puce sont recouvertes d'une plaquette métallique (22, 24), qui est pourvue de trous (26, 28) en alignement respectivement avec l'entrée (12) et avec la sortie (14), tandis qu'il est prévu, entre la face de la puce et les plaquettes métalliques (22, 24), des couches de silicone (30, 32), qui comportent des évidements (34, 36) en alignement respectivement avec l'entrée (12) et avec la sortie (14).

2. Pompe implantable à perfusion selon la revendication 1, **caractérisée en ce qu'**une pluralité de chemins d'écoulement de fluide, représentant une résistance différente par étranglement, sont formés sur la puce, l'entrée (12) étant disposée de manière centrale et les sorties (14) des chemins individuels d'écoulement de fluide étant disposées à symétrie ponctuelle par rapport à l'entrée (12).
